# FASCICULE DE BREVET EUROPEEN

(11) **EP 1 313 855 B1**
(45) Date de publication et mention de la délivrance du brevet: **31.05.2006**
(21) Numéro de dépôt: 01967422.5
(22) Date de dépôt: 03.09.2001
(51) Int. Cl.: C12N 15/12, C07K 14/47, C07K 16/18, A61K 38/17, A61K 31/713, G01N 33/574

(54) **PROTEINE BARD1 TRONQUEE, ET SES APPLICATIONS DIAGNOSTIQUES ET THERAPEUTIQUES**
VERKÜRZTES BARD1 PROTEIN UND DESSEN DIAGNOSTISCHE UND THERAPEUTISCHE ANWENDUNGEN
TRUNCATED BARD1 PROTEIN, AND ITS DIAGNOSTIC AND THERAPEUTIC USES

(30) Priorité: 01.09.2000 FR 0011207
(43) Date de publication de la demande: 28.05.2003
(73) Titulaire: INSTITUT NATIONAL DE LA SANTE ET DE LA RECHERCHE MEDICALE (INSERM), 75013 Paris (FR); UNIVERSITE DE GENEVE, 1211 Genève 4 (CH)
(72) Inventeur: GAUTIER, Fabien, F-49240 Avrille (FR); HARB, Jean, F-44100 Nantes (FR); MEFLAH, Khaled, F-44000 Nantes (FR); IRMINGER-FINGER, Irmgard, CH-1207 Genève (CH)
(74) Mandataire: Bernasconi, Jean Raymond
(86) Numéro de dépôt international: PCT/FR2001/002731
(87) Numéro de publication internationale: WO 2002/018536

(56) Documents cités:
- WO-A-98/12327
- WU L C ET AL: "IDENTIFICATION OF A RING PROTEIN THAT CAN INTERACT IN VIVO WITH THEBRCA1 GENE PRODUCT" NATURE GENETICS,US,NEW YORK, NY, vol. 14, no. 4, 1 décembre 1996 (1996-12-01), pages 430-440, XP002050113 ISSN: 1061-4036 cité dans la demande
- TO HOA THAI ET AL: "Mutations in the BRCA1-associated RING domain (BARD1) gene in primary breast, ovarian and uterine cancers" HUMAN MOLECULAR GENETICS,GB,OXFORD UNIVERSITY PRESS, SURREY, vol. 7, no. 2, 1998, pages 195-202, XP002127132 ISSN: 0964-6906
- GAUTIER FABIEN ET AL: "Identification of an apoptotic cleavage product of BARD1 as an autoantigen: A potential factor in the antitumoral response mediated by apoptotic bodies." CANCER RESEARCH, vol. 60, no. 24, 15 décembre 2000 (2000-12-15), pages 6895-6900, XP002167899 ISSN: 0008-5472

## Description

La présente invention a trait à un nouveau polypeptide issu du clivage de la protéine BARD1, et à ses applications diagnostiques et thérapeutiques.

La protéine BARD1 est une protéine de 97 kD qui interagit avec le produit du gène supresseur de tumeur BRCA1, par le biais de motifs en anneaux présents sur BRCA1 et BARD1 (pour "BRCA1 Associated Ring Domain") (Wu et al, 1996). Le gène codant pour cette protéine a été récemment cloné (WO98/12327).

Boisteau et al. décrit que les corps apoptotiques dérivés des cellules PROb de carcinome de rat ont un effet anti-tumoral moyennant la production de anticorps. Cet effet n'est pas observé avec des protéines extraites de cellules PROb entières. Ce document divulgue aussi une analyse Western Blot où lesdits anticorps reconnaissent une bande à approximativement 70 kD dans les corps apoptotiques et une bande à approximativement 100 kD dans les cellules PROb entières.

Les auteurs de la présente invention ont maintenant identifié un nouveau polypeptide qui correspond à un fragment protéolytique de BARD1, clivée au cours du processus d'apoptose.

Cette protéine tronquée est très immunogène et peut être utilisée entre autres dans le traitement des cancers ou dans le suivi de l'efficacité du traitement par des drogues pro-apoptotiques de patients atteints de cancer.

L'invention a donc pour objet ce polypeptide isolé qui possède un poids moléculaire d'environ 67 kD, tel que mesuré par exemple par électrophorèse dans des conditions dénaturantes (SDS-PAGE), et dont la séquence est constituée par la séquence d'acides aminés de la protéine BARD1 délétée de sa partie N-terminale qui comprend le domaine RING.

Plus particulièrement, le polypeptide de l'invention peut être défini comme étant constitué par environ 505 à 525 acides aminés à partir de l'extrémité C-terminale de la protéine BARD1 humaine (dont la séquence connue est présentée en annexe SEQ ID n° 1). Plus particulièrement encore, le polypeptide de l'invention est constitué de 525 à 522 acides aminés C-terminaux de BARD1 humaine.

Le polypeptide de l'invention peut être par exemple purifié à partir des corps apoptotiques issus de lignées cellulaires de carcinomes mammaires ou de colon.

Plus particulièrement, le polypeptide de l'invention peut être obtenu par le procédé consistant à :
- cultiver jusqu'à confluence des cellules, par exemple appartenant à une ligné cellulaire telle que PROb, SW48 ou MCF7 ;
- induire l'apoptose de ces cellules en les traitant avec un milieu de culture contenant du butyrate de sodium (NaB) 5 mM, à 37°C pendant 24 heures ;
- ajouter de la protéine recombinante BARD1 isolée ;
- incuber pendant un temps suffisant, par exemple 60 minutes à 37°C, pour observer le clivage de la protéine BARD1 en une forme de 67 kDa.

Le polypeptide de l'invention est reconnu par un anticorps dirigé contre un polypeptide correspondant aux acides aminés 255 à 265 de BARD1.

Les auteurs de l'invention ont par ailleurs montré que l'hydrolyse de BARD1 intervient à un stade précoce de l'apoptose et d'une manière dépendante du cycle cellulaire. Cette hydrolyse est inhibée par l'EGTA et l'inhibiteur de calpaïne 1, la N-acétyl-leu-leu-norleucinal (ALLnL) mais pas par plusieurs inhibiteurs de caspases, ce qui suggère une hydrolyse par des cysteine protéases dépendantes du calcium, les calpaïnes.

Est également comprise dans la définition de ce polypeptide de 67 kD toute protéine constituée d'une séquence homologue à ladite séquence de 505 à 525 acides aminés.

L'invention a également pour objet un acide nucléique codant pour cette protéine tronquée ou ses homologues.

Par séquence d'acides aminés homologue, on entend une séquence similaire à au moins 70%, de préférence 80%, de préférence encore 90% de ladite séquence de 505 à 525 acides aminés.

Le terme "similaire" se réfère à la ressemblance parfaite ou identité entre les acides aminés comparés mais aussi à la ressemblance non parfaite que l'on qualifie de similitude. Cette recherche de similitudes dans une séquence polypeptidique prend en compte les substitutions conservatives qui sont des substitutions d'acides aminés de même classe, telles que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires. (tels que la glycine, l'alanine, la valine, la leucine, l'isoleucine, la proline, la phénylalanine, la méthionine, le tryptophane, et la cystéine).

Plus généralement, par "séquence d'acides aminés homologue", on entend donc toute séquence d'acides aminés qui diffère de ladite séquence par substitution, délétion et/ou insertion d'un acide aminé ou d'uri nombre réduit d'acides aminés, notamment par substitution d'acides aminés naturels par des acides aminés non naturels ou pseudoacides aminés à des positions telles que ces modifications ne portent pas significativement atteinte aux propriétés biologiques évoquées ci-dessus.

L'homologie est généralement déterminée en utilisant un logiciel d'analyse de séquence (par exemple, Sequence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (i.e. identité ou similitude, comme défini plus haut). A cette fin, il peut être nécessaire d'introduire de manière artificielle des espaces (" gaps ") dans la séquence. Une fois l'alignement optimal réalisé, le degré d'homologie est établi par enregistrement de toutes les positions pour lesquelles les acides aminés des deux séquences comparées sont identiques, par rapport au nombre total de positions.

Le polypeptide de la présente invention peut être synthétisé par toutes les méthodes bien connues de l'homme du métier. Le polypeptide de l'invention peut par exemple être synthétisé par les techniques de la chimie de synthèse, telles que la synthèse de type Merrifield qui est avantageuse pour des raisons de pureté, de spécificité antigénique, d'absence de produits secondaires non désirés et pour sa facilité de production.

La protéine produite peut ensuite être récupérée et purifiée.

Les procédés de purification utilisés sont connus de l'homme du métier. Le polypeptide recombinant obtenu peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps mono- ou polyclonaux spécifiques, etc.

Le polypeptide de l'invention peut en particulier être purifié à partir de corps apoptotiques provenant de cellules tumorales par chromatographie d'affinité sur une colonne d'anticorps spécifiques de l'extrémité C terminale de la protéine BARD1.

La séquence d'acide nucléique codant pour la protéine BARD1 tronquée, peut être insérée dans un vecteur d'expression, dans lequel elle est liée de manière opérante à des éléments permettant la régulation de son expression, tels que notamment des promoteurs, activateurs et/ou terminateurs de transcription.

Une protéine recombinante peut alors également être produite par un procédé, dans lequel un vecteur contenant un acide nucléique tel que défini ci-dessus est transféré dans une cellule hôte qui est mise en culture dans des conditions permettant l'expression du polypeptide correspondant.

Les signaux contrôlant l'expression des séquences nucléotidiques (promoteurs, activateurs, séquences de terminaison...) sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standard, telles que par exemple l'électroporation ou la précipitation au phosphate de calcium.

Les vecteurs de clonage et/ou d'expression tels que décrits ci-dessus, comprenant une séquence nucléotidique définie selon l'invention font également partie de la présente invention.

L'invention vise en outre les cellules hôtes transfectées, de manière transitoire ou stable, par ces vecteurs d'expression. Ces cellules peuvent être obtenues par l'introduction dans des cellules hôtes, procaryotes ou eucaryotes, d'une séquence nucléotidique insérée dans un vecteur tel que défini ci-dessus, puis la mise en culture desdites cellules dans des conditions permettant la réplication et/ou l'expression de la séquence nucléotidique transfectée.

Les propriétés de la protéine BARD1 tronquée selon l'invention ou de l'acide nucléique codant pour cette protéine comme répresseur de tumeurs peuvent être mises à profit dans le traitement des tumeurs. Il peut s'agir de tout type de tumeur, mais plus particulièrement des cancers du sein, de l'ovaire, du poumon ou du tractus digestif, tels que les carcinomes du colon.

L'invention a donc également pour objet une composition pharmaceutique comprenant un polypeptide tel que défini précédemment ou un acide nucléique codant pour ledit polypeptide, en association avec un véhicule pharmaceutiquement acceptable.

Les modes d'administration, les posologies et les formes galéniques des compositions pharmaceutiques selon l'invention, contenant au moins un polypeptide, peuvent être déterminées de manière usuelle par l'homme du métier, notamment selon les critères généralement pris en compte pour l'établissement d'un traitement thérapeutique adapté à un patient, comme par exemple l'âge ou le poids corporel du patient, la gravité de son état général, la tolérance au traitement, et les effets secondaires constatés, etc.

De manière générale, une quantité thérapeutiquement ou prophylactiquement efficace variant d'environ 0,1 µg à environ 1 mg peut être administrée à des adultes humains.

L'invention a également pour objet une composition pharmaceutique comprenant un acide nucléique tel que défini précédemment, codant pour la protéine BARD1 tronquée et un véhicule pharmaceutiquement acceptable, ladite composition étant destinée à être utilisée en thérapie génique. L'acide nucléique de préférence inséré dans un vecteur généralement viral (tel que les adénovirus et les rétrovirus) peut être administré sous forme nue, exempt de tout véhicule favorisant le transfert à la cellule cible, tels que des liposomes anioniques, des lipides cationiques, des microparticules, par exemple des microparticules d'or, des agents de précipitation, par exemple du phosphate de calcium, ou tout autre agent facilitant la transfection. Dans ce cas, le polynucléotide peut être simplement dilué dans une solution physiologiquement acceptable, telle qu'une solution stérile ou une solution stérile tampon, en présence ou en l'absence d'un véhicule.

De manière alternative, un acide nucléique de l'invention peut être associé à des agents qui facilitent la transfection. Il peut être, entre autres, (i) associé à un agent chimique qui modifie la perméabilité cellulaire tel que la bupivacaïne ; (ii) encapsulé dans des liposomes, éventuellement en présence de substances supplémentaires facilitant la transfection ; ou (iii) associé à des lipides cationiques ou des microparticules de silice, d'or ou de tungstène.

Lorsque les constructions d'acide nucléique de l'invention recouvrent des microparticules, celles-ci peuvent être injectées par voie intradermique ou intraépidemnique par la technique du canon à gènes, "gene gun" (WO 94/24263).

La quantité à utiliser comme médicament dépend notamment de la construction d'acide nucléique elle-même, de l'individu auquel cet acide nucléique est administré, du mode d'administration et du type de formulation, et de la pathologie. De manière générale, une quantité thérapeutiquement ou prophylactiquement efficace variant d'environ 0,1 µg à environ 1 mg, de préférence d'environ 1 µg à environ 800 µg et, de manière préférentielle d'environ 25 µg à environ 250 µg, peut être administrée à des adultes humains.

Les constructions d'acides nucléiques de l'invention peuvent être administrées par toute voie d'administration conventionnelle telle que notamment par voie parentérale. Le choix de la voie d'administration dépend en particulier de la formulation choisie. Une administration ciblée au site des tumeurs visées peut être particulièrement avantageuse.

L'invention a donc enfin pour objet l'utilisation de la dite composition pharmaceutique pour la fabrication d'un medicament destiné au traitement des tumeurs.

Le patient visé est généralement un être humain, mais l'application peut également être étendue à tout mammifère le cas échéant.

L'apparition de la forme tronquée de BARD1 apparaissant lors des processus d'apoptose rend par ailleurs possible le suivi *in vitro* de l'efficacité d'un traitement anti-cancéreux chez un patient traité avec des drogues pro-apoptopiques. Pour cela on peut mettre en oeuvre un procédé de détection *in vitro* du polypeptide de 67 kD de l'invention dans un échantillon biologique, tel qu'un échantillon de tissus tumoraux.

Selon une variante, on peut mettre en oeuvre un procédé de détection *in vitro* d'anticorps produits contre le polypeptide de 67 kD immunogène, dans un échantillon biologique, tel qu'un échantillon de sang ou d'urine des patients.

Ces procédés peuvent faire appel aux techniques usuelles d'immunodétection, telles que le Western Blot ou l'immunohistochimie par exemple, à l'aide d'un anticorps anti-BARD1 ou anti-BARD1 tronquée, lorsqu'il s'agit de détecter le polypeptide de 67 kD, ou à l'aide dudit polypeptide ou d'un fragment épitopique de celui-ci, lorsqu'il s'agit de détecter des anticorps.

Les exemples et figures suivants illustrent l'invention sans en limiter la portée.

### LEGENDE DES FIGURES

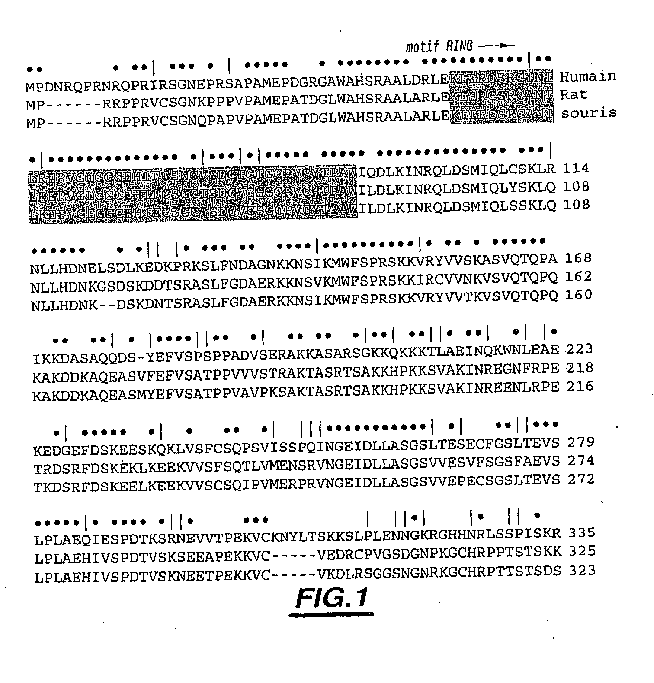

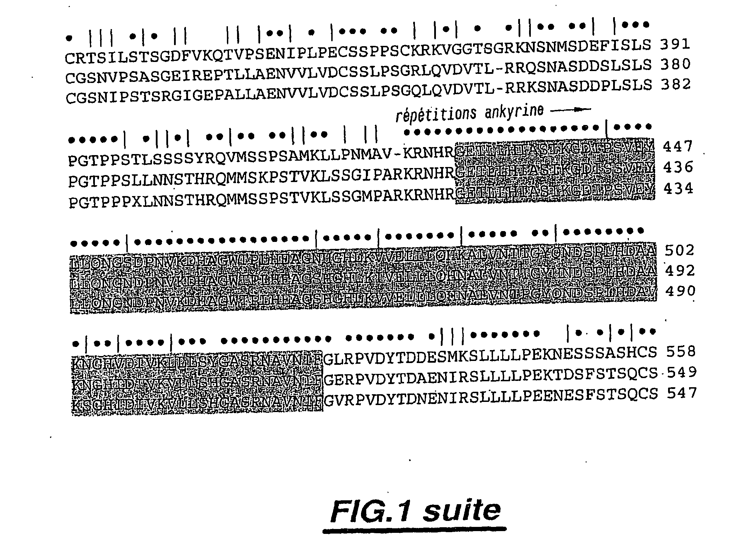

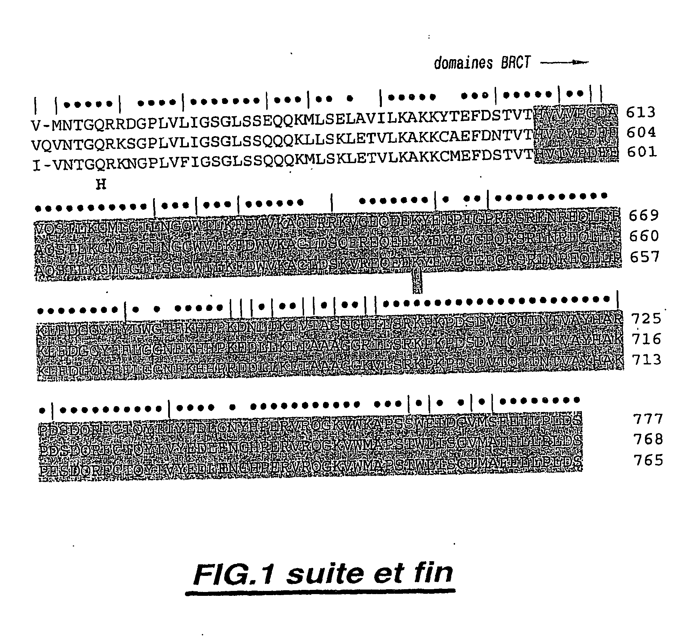

La figure 1 présente un alignement des séquences en acides aminés des protéines BARD1 d'homme, de rat et de souris. Les séquences correspondantes au motif RING, aux trois répétitions ankyrine et aux deux domaines BRCT en tandem sont soulignées. La mutation Q564H de la protéine humaine BARD1 conservée est aussi indiquée. Les séquences sont alignées en introduisant des espaces ("gaps") pour atteindre le maximum d'identité des séquences d'acides aminés. Les valeurs pour l'identité en acides aminés sont ensuite calculées en considérant chaque espace comme une unique dissemblance (tableau 1). La séquence d'ADNc de rat de BARD1 est disponible sur EMBL sous le numéro d'accès AF182946, celle de souris sous le numéro AF057157 et celle d'homme sous le numéro Genbank U76638.

La figure 2A représente une analyse Western blot de protéines de cellules de carcinomes de rats et d'hommes et de leurs corps apoptotiques révélées avec un anticorps monoclonal 6D10 anti-BARD1 de rat.

La figure 2B représente le même type d'analyse, avec une révélation par un anticorps polyclonal anti-humain 669D. A noter que a.b. signifie corps apoptotique.

La figure 2C représente une analyse Western Blot de protéines de corps apoptotiques, d'abord réalisée en utilisant un anticorps polyclonal anti BARD1 de souris WFS. Les complexes immuns sont dissociés en utilisant le kit chemicon et le filtre est soumis de nouveau à un anticorps anti-BARD1 humaine 669D.

La figure 2D représente une immunoprécipitation de lysats de corps apoptotiques dérivés de plusieurs cellules de carcinome avec une révélation par un anticorps anti-BARD1 humaine 669D. Le précipitat a été soumis à une électrophorèse puis un immunoblot a été réalisé avec des sérums de rats immunisés avec des corps apoptotiques PROb, dilués au 1/250^{ème}.

La figure 3A représente une analyse des produits d'hydrolyse *in vitro* de la protéine BARD1 humaine par des lysats cellulaires SW48 traités à confluence par 5 mM de butyrate de sodium pendant 24 heures. Les corps apoptotiques recueillis dans le surnageant (1) ou les cellules adhérentes (2) ont été solubilisés dans un tampon DIV et ajoutés à une protéine BARD1 humaine marquée à la méthionine ³⁵S. Après 4 heures d'incubation à 37°C, les produits d'hydrolyse ont été séparés par SDS-PAGE et auto-radiographiés en utilisant du phosphorimager.

La figure 3B représente le même type d'analyse, les cellules adhérentes étant solubilisées dans du tampon DIV et incubées avec une protéine BARD1 humaine marquée à la méthionine ³⁵S pendant 0 à 3 heures respectivement (TO à T3).

La figure 4 est un graphe représentant la croissance de tumeurs PROb dans les rats BDIX après vaccination par le fragment F1 de BARD1 ou une protéine contrôle (fucosyl transférase).

### EXEMPLES

### Exemple 1 :

### Identification d'une protéine de 67 kD comme produit de clivage de la protéine BARD1 :

### Matériels et méthodes :

### Cultures Cellulaires

Les cellules de carcinome de colon de rat REGb et d'adénocarcinome de colon de rat PROb utilisées sont dérivées d'une lignée cellulaire induite par la diméthylhydrazine (Caignard et al, 1985). Le carcinome mammaire de rat 13762, le carcinome de colon humain SW48 et le carcinome mammaire MCF7 ont été obtenus auprès du ECACC. Les cellules ont été cultivées en cultures mono-couche à 37°C dans du milieu RPMI1640 (Gibco) avec 10% de sérum de veau foetal et 2mM de glutamine. Les cellules ont été passées avec 0,025% de trypsine et 0,02% d'EDTA.

### Criblage immunologique et Clonage de l'ADNc de BARD1 de rat

Une banque d'ADNc de la lignée cellulaire de carcinome de rat PROb a été construite dans le vecteur d'expression λTriplEx (Clontech). Un million de plages ont été criblées avec des sérums de rats vaccinés par des corps apototiques et de l'IL-2 (Boisteau et al 1997). Les anticorps contre *E.coli* ont été éliminés de l'antisérum de rats par incubation des bactéries E. coli soniquées avec du sérum dilué au 1/10^{ème} dans du PBS plus 5% de lait écrémé déshydraté pendant 4 heures à température ambiante, puis centrifugés à 13,000 g pendant 10 minutes. L'insert de 456 paires de bases (fragment F1) a été séquencé et la séquence a été soumise à une analyse sur la banque de gènes NCBI. Elle représentait une forte homologie avec la protéine BARD1 humaine. Le clonage de l'ADNc complet de BARD1 de rat a été effectué en utilisant la banque d'ADNc PROb construite par le kit PCR SMART de Clontech. Les amorces internes pour la RACE PCR ont été choisies à partir de l'insert cloné selon les instructions du fabricant.

### Clonage de l'ADNc BARD1 humain

Trois fragments d'ADNc BARD1 humain ont été amplifiés à partir d'ARN total extrait de lignées cellulaires de carcinomes de colon humain SW48. Les fragments, désignés A,B et C, ont été obtenus en utilisant les amorces suivantes : fragment A amorce sens R135S/ amorce anti-sens B202N (Thai et al, 1998), fragment B, amorce sens B202A (Thai et al, 1998) amorce anti-sens : 5' CACCAATGCCTTATGCTGGAGC 3'; fragment C, amorce sens : 5' GAAGTAGTGACTCCTGAGAAGG 3'/amorce antisens 5' TCAGCTGTCAAGAGGAAGCAACTC 3'. Chaque fragment a été cloné dans un plasmide de pGEM (Promega) puis excisé en utilisant Not1-Pst1/Pst1-Hind III/ Hind III-Bst XI, respectivement purifié, puis ligaturé dans les sites Not1/Bst XI de pGEM.

### Induction apoptotique et purification des corps apoptotiques.

L'apoptose a été induite par un traitement au butyrate de sodium (NaB). Les cellules à différents stades de confluence ont été traitées dans du milieu complet à 37°C avec 5mM de NaB (Sigma) pendant différentes périodes. Les corps apoptotiques ont été purifiés comme décrit précédemment (Gautier et al, 1999):

### Production et Purification des Fragments F1 de BARD1 de rat

Le fragment F1 de BARD1 de rat a été excisé du plasmide λ Trip1ex de la banque d'ADNc et inséré en cadre dans le site Pst1 du plasmide pQE32 (Qiagen). La protéine de fusion résultante, contenant un tag 6xHis placé à l'extrémité N-terminale du fragment F1 de BARD1, a été exprimée dans *E. coli* puis purifiée par chromatographie d'affinité sur une résine *Ni-NTA* en utilisant les recommandations des fabricants pour le kit QIA expressionnist (Qiagen).

### Immunisation des souris et production d'un anticorps monoclonal

Des souris Balb-c (Iffa-credo) ont reçu des injections sous-cutanées de 100 µg du fragment F1 de BARD1 de rat dans 0,1ml d'adjuvant incomplet de Freund (Life Technologies) émulsifié dans 0,1 ml de tampon PBS stérile, 0,5% de Triton X-100, à des intervalles de 3 semaines. Les spiénocytes d'une souris ont été fusionnés avec le myélome de souris SP20 (ECACC) en présence de polyéthylèneglycol 1500 (Boehringer Mannheim). Les hybridomes ont été déposés sur des plaques à 96 puits dans du milieu complet supplémenté avec 20% de sérum de veau foetal, de l'hypoxanthine-aminoptérine-thymidine (Sigma) et 1,5 ng/ml d'IL6 recombinante (RD Systems). Les sumageants des hybridomes ont été testés par ELISA en utilisant un fragment F1 de BARD1 purifié comme antigène.

### Immunoprécipitation

Les corps apoptotiques ont.été extraits sur de la glace avec 2% de Triton X-100 dans du PBS supplémenté avec un cocktail d'inhibiteurs de protéases exempt d'EDTA (Boehringer Mannheim) pendant 30 minutes. L'extrait était centrifugé pendant 15 minutes à 13, 000 g et le surnageant était incubé avec des anticorps polyclonaux de lapin dirigés contre la protéine BARD1 humaine (669D) (Wu et al, 1996 et Jin et al, 1997) dilués au 1/1000^{ème}. Après 4 heures d'incubation, avec une agitation constante, les complexes immuns ont été immunoprécipités par addition de 50 µl d'agarose anti-IgG de lapin. Les complexes immuns liés à l'agarose ont été lavés avec du PBS à 1% de Triton X-100 contenant des inhibiteurs de protéases et ont été extraits des billes d'agarose par chauffage dans un tampon de réduction pour une électrophorèse et un immunoblot comme décrit ci-après.

### Western Blot

L'électrophorèse a été menée dans des conditions dénaturantes (SDS PAGE) (Laemmli et al, 1970). Les protéines ont été transférées sur un filtre de PVDF 0,45 µm (Millipore) et mises en contact avec des anticorps primaires. Les anticorps secondaires conjugués à la peroxidase de raifort ont été utilisés, dilués au 1/15000 (Sigma). Les complexes immuns ont été visualisés par chimioluminescence en utilisant un kit Super Signal (Pierce).

### Transcription/Traduction in vitro couplées et détermination du clivage de la protéine in vitro :

La protéine BARD1 humaine marquée par la méthionine ³⁵S a été transcrite et traduite *in vitro* en utilisant le kit systems de lysat de réticulocytes couplé au TNT (Promega). 1 µg de plasmide a été utilisé dans un milieu réactionnel pour la transcription et la traduction, contenant 4 µl de méthionine ³⁵S (NEN). Pour le clivage *in vitro,* 2 µl de produits de transcription/traduction ont été incubés avec des extraits cellulaires apoptotiques ou non-apoptotiques préparés dans un tampon DIV (20mM HEPES pH 7,5, 10 mM NaCl, 1,5 mM MgCl₂, 0.1% SB14, 0,5 mM PMSF) à 37°C pendant la période de temps indiquée. Les produits d'hydrolyse ont été ensuite séparés par SDS-PAGÉ et révélés par autoradiographie en utilisant le phosphorimager 445SI (Molecular Dynamics). L'inhibition du clivage a été évaluée en ajoutant des inhibiteurs de caspases ou un inhibiteur de protéasome (lactacystine) (Calbiochem) ou l'inhibiteur de calpaïne l (ALLnL) (Chemicon).

### Synchronisation de cycles cellulaires

Les cellules SW48 ont été arrêtées dans G₀ par inhibition de contact dans des fioles de 175 cm². Après 3 jours de confluence, les cellules ont été divisées à 1:10 dans les fioles de 75 cm² et à une concentration de 3 x 10⁶ cellules par fiole. 12, 20, 28 36 et 44 heures après l'ensemencement, les cellules ont été traitées avec 5 mM de NaB pendant 24 heures et recueillies. Pour déterminer la distribution du cycle cellulaire à chaque temps, les contenus de chaque fiole ont été soumis à une trypsination, lavées trois fois dans 10ml de PBS glacé, et fixées avec 1 ml d'éthanol glacé à 70% ajouté goutte à goutte, pendant 16 heures à -20°C. Les cellules fixées ont été mises en culot, resuspendues dans 500 µl de tampon PC (96% 0.2M Na₂HPO₄, 4% 0.1 M acide citrique pH 7,8) et laissées 30 minutes à température ambiante. Ensuite elles ont été lavées et re-suspendues dans 500 µl d'iodure de propidium dans une solution de coloration (PBS, 0,12 % Triton X-100, 0,12 mM EDTA, 100 µg/ml RNase A), incubées pendant 30 minutes à 37°C et analysées sur un cytomètre de flux FACScan (Beckton Dickinson). Pour la détermination du clivage de la protéine *in vitro,* les cellules ont été grattées et les extraits cellulaires ont été préparés comme décrits précédemment.

### Résultats

### Clonage de l'ADNc codant pour la protéine 67 kD

L'immunocriblage de la banque d'ADNc λTrip1Ex avec des sérums de rat traités pour un carcinome avec des corps apoptotiques/IL-2, a conduit à l'identification d'un insert positif de 456 paires de bases du gène BARD1 de rat (Fragment F1). Ce fragment s'étend entre les acides aminés 460 à 611 (figure 1). Le tableau 1 ci-après présente le pourcentage d'homologie entre la protéine BARD1 de rat, d'homme et de souris.

### Pourcentage d'homologie entre la protéine BARD1 de rat, d'homme et de souris

| Espèce | Total | RING | Ankyrine | BRCT |
|---|---|---|---|---|
| | | | | |
| rat/souris | 87.9 | 95.5 | 93.9 | 94.2 |
| rat/humain | 64.8 | 86.6 | 90.9 | 80.3 |
| souris/humain | 67.5 | 86.6 | 90.9 | 79.7 |

### La protéine de 67 kD est un fragment de BARD1

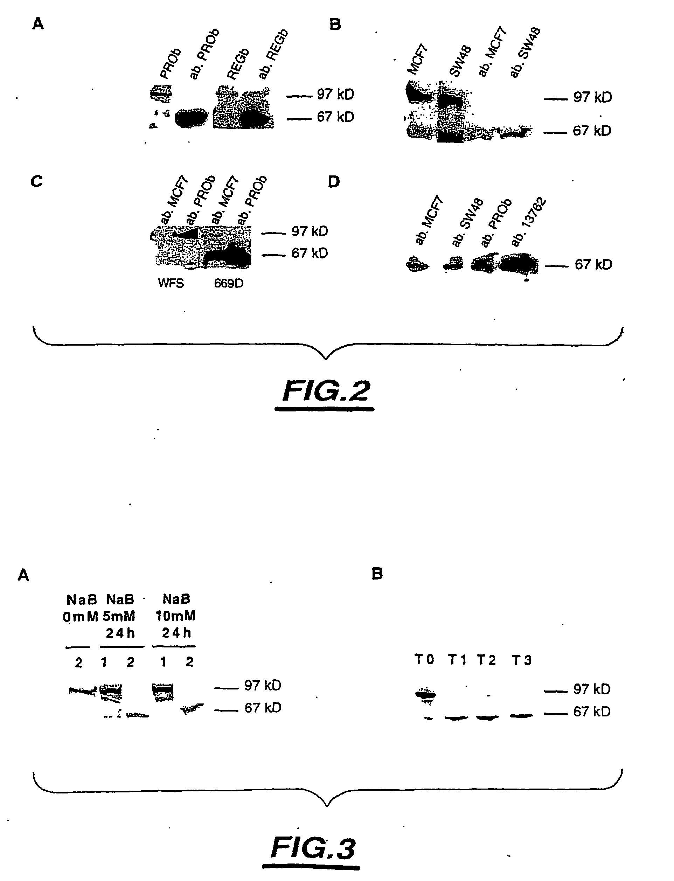

Afin de pouvoir prouver l'identité de la protéine de 67kD, comme un fragment de BARD1, les auteurs de l'invention ont déterminé son expression dans les cellules tumorales et les corps apoptotiques. A cette fin, ils ont produit des anticorps monoclonaux (clone 6D10) contre le fragment F1. Lorsqu'il a été testé sur des cellules PROb et REGb de carcinome de rat, cet anticorps monoclonal 6D10 a reconnu une protéine de 97 kD mais également une bande à approximativement 67 kD dans les corps apoptotiques dérivés de ces cellules après traitement au butyrate de sodium (figure 2A). Ce résultat a été confirmé en utilisant un anticorps polyclonal 669D (Wu et al, 1996 et Jin et al, 1997) contre la protéine BARD1 humaine. Un traitement des cellules de colon humain SV48 ou MCF7 de glande mammaire avec du butyrate de sodium a conduit à un résultat similaire après un immunoblot avec un anticorps polyclonal 669D (figure 2B). Finalement, l'immunoprécipitation de BARD1 à partir de corps apoptotiques dérivés de carcinomes humains de rats avec l'anticorps polyclonal 669D suivi d'un immunoblot avec un sérum de rat soigné a permis de détecter une protéine de 67 kD (figure 2D). L'ensemble de ces résultats prouve l'identité de la protéine de 67 kD comme un fragment de BARD1.

### BARD1 est clivée durant l'apoptose

Lorsque les corps apoptotiques, issus soit de carcinomes MCF7 humains soit de carcinome PROb de rat ont été incubés sur la même membrane successivement avec l'anticorps polyclonal anti-BARD1 humaine 669D ou l'anticorps WFS contre la partie N-terminale de la protéine BARD1 de souris (acides aminés 101-114) (Irminger-Finger 1988), les auteurs de l'invention ont observé que l'anticorps WFS ne reconnaissait pas la molécule 67 kD tandis que l'anticorps 669D reconnaissait la molécule 67 kD dans chacun des types de corps apoptotiques (figure 2C). Ceci suggère fortement que le site de clivage de BARD1 est localisé dans la partie N-terminale mais en aval du domaine RING (acides aminés 40-84, figure 1) essentielle pour l'interaction des protéines BARD1 et BRCA1 (Wu et al, 1996).

### Le clivage de BARD1 durant l'apoptose est dépendant du cycle cellulaire.

Les auteurs de l'invention ont examiné l'effet du traitement au NaB pour le clivage de BARD1 dans les cellules SW48 adhérentes ou dans les corps apoptotiques recueillis à partir du surnageant. La figure 3A montre que les lysats des cellules adhérentes clivent complètement la protéine BARD1 humaine radiomarquée après 4 heures d'incubation, puisque la protéine BARD1 humaine de pleine longueur disparaît complètement et une protéine 67 kD apparaît. Cependant, l'incubation avec des lysats de corps apoptotiques n'a pas d'effet sur l'hydrolyse de hBARD1. Ces résultats indiquent que l'activité protéolytique impliquée dans le clivage de BARD1 intervient avant l'étape ultime d'apoptose qui conduit à la formation de corps apoptotiques et au détachement cellulaire. L'analyse des cinétiques conduisant à l'hydrolyse de hBARD1 avec des lysats des cellules adhérentes SW48 traitées pendant 48 heures avec 5mM de NaB a montré que le clivage est terminé au bout d'une heure (figure 3B). Une analyse complémentaire de l'induction cinétique de ce clivage par le NaB a montré que la protéine p67 apparaissait après 4 heures de traitement des cellules par le NaB et que le clivage était pratiquement complet au bout de 12 heures. De manière intéressante, l'hydrolyse de l'hBARD1 est régulée de manière dépendante du cycle cellulaire, avec une prédominance au cours de la phase G₀/G₁. Ceci a été démontré par addition de NaB 32 heures après l'étalement des cellules ; stade auquel 80% des cellules étaient en phase G₀/G₁ et seulement 5% en phase G2/M, ce qui a conduit à une conversion complète de hBARD1 en p67.

### Exemple 2 :

### Clivage par des calpaïnes :

### Matériel et Méthodes

### Détermination de l'activité caspase

Pour les dosages de l'activité caspase, 10 µg d'extraits cellulaires ont été dilués dans 5 µl de tampon DIV :
L'acétyl-Asp-Glu-Val-Asp-7-amino-4 méthylcoumarine (Ac-DEVD-AMC) l'acétyl-Val-Glu-lle-Asp-7-amino-4-méthylcoumarine (Ac-VEID-AMC) et l'acétyl-lle-Glu-lle-Asp-7-amino-4-méthylcoumarine (Ac-IETD-AMC) (Bachem), substrats des caspases 3,6 et 8, respectivement, ont été ajoutées à une concentration finale de 50 µM. L'activité de clivage a été contrôlée sur Fluorolite 1000 (Dynatech laboratories).

### Fraction Cellulaire

Les cellules ont été cultivées dans des fioles de 75 cm², trypsinisées et re-suspendues dans 100 µl de tampon CEB (50mM HEPES pH 7,4 50 mM M-gCl₂, 1mM DTT, -10 µM Cytochalasine B). Les cellules resuspendues ont été laissées sur de la glace pendant 30 minutes et puis homogénéisées par 50 coups d'homogénisateur Dounce, refroidi dans la glace. La fraction nucléaire a été préparée par centrifugation à 800 x g pendant 10 minutes à 4°C. Le culot a été resuspendu dans un tampon CEB et stocké à -80°C. Les fractions mitochondriales et post-mitochondriales ont été obtenues après centrifugation à 13 000 x g pendant 10 minutes à 4°C. A la fois, le culot mitochondrial resuspendu dans CEB et la fraction post-mitochondriale ont été divisés en fractions aliquotes et conservés à -80°C.

### Résultats

Pour définir l'activité protéolytique responsable du clivage de h-BARD1, différents préparations d'extraits cellulaires à partir des cellules SW48 traitées avec NAB ont été testées. Il apparaît qu'à la fois les préparations nucléaires et les préparations mitochondriales étaient capables de cliver hBARD1. Le surnageant à 13,000 x g de la préparation d'organelle n'avait pas d'effet.

La cascade de protéases effectrices du processus apoptotique comprend des cystéines protéases telles que des caspases ou des calpaïnes. Les auteurs de l'invention ont alors déterminé les activités de caspases lors du traitement des cellules SW48 avec NaB, en utilisant des substrats spécifiques, et trouvé que les activités des caspases 3, 6, et 8 augmentaient progressivement, la caspase 3 étant la plus active après 6 heures et 12 heures de traitement NaB. L'utilisation d'inhibiteurs peptidiques pour les caspases a montré que la benzyloxycarbonyl-Val-Ala-Asp-fluorométhylcétone (Z-VAD-fmk), un inhibiteur des caspases, inhibait légèrement l'hydrolyse de hBARD1 à forte concentration (100 µM). En revanche, les inhibiteurs spécifiques de la caspase 3, la benzyloxycarbonyl-Asp-Glu-Val-Asp-fluorométhylcétone (Z-DEVD-fmk) ou de la caspase 6, la benzyloxycarbonyl-Val-Glu-Ile-Asp-fluorométhylcétone (Z-VEID-fmk) n'avaient pas d'effet. Ce phénomène a été confirmé par le fait que la caspase 3 purifiée n'avait pas d'effet sur l'hydrolyse de h-BARD1. Ces résultats montrent clairement que h-BARD1 n'est pas un substrat direct de caspases. De plus la lactacystine, inhibiteur de protéasome, ne bloque pas la protéolyse de hBARD1 même à des concentrations de 100 µM, ceci excluant que le protéasome puisse être impliqué dans ce mécanisme.

Un certain nombre de protéines qui sont dégradées durant l'apoptose sont des cibles des calpaïnes. Un mécanisme possible pour l'activation des calpaïnes implique le clivage d'un inhibiteur de calpaïne *in vivo,* la calpastatine (DeMartino et al, 1984). Les résultats des auteurs de l'invention montrent premièrement que la calpastatine était complètement clivée dans les cellules SW48 après 12 heures de traitement au NaB et deuxièmement que l'inhibiteur I de calpaïne ALLnL et l'EGTA, inhibaient fortement l'hydrolyse de hBARD1 d'une manière dose dépendante. L'ensemble de ces résultats suggère fortement que la protéine BARD1 est hydrolysée par des calpaïnes.

### Exemple 3 :

### Purification de la protéine BARD1 clivée

### Production d'anticorps spécifiques de la protéine tronquée

Des anticorps poly et monoclonaux dirigés contre des séquences peptidiques situées aux extrémités N et C terminales de la protéine tronquée sont produits:
- contre le peptide 1 correspondant aux acides aminés 255 à 265 de la protéine BARD1 humaine ;
- contre le peptide 2 correspondant aux acides aminés 527 à 540 de la protéine BARD1 humaine.

Les peptides ont été couplés à la KLH. Chaque lapin a reçu trois injections de 200 µg de chaque peptide, espacées de 15 jours. Une quatrième injection a été faite trois semaines après la dernière injection. La production d'anticorps a été testée sur le sérum des lapins par la technique d'ELISA en utilisant comme antigène, les peptides libres.

Les titre des sérums obtenus sont élevés (1/16000). De plus, aucune réactivité croisée n'a été observée entre les sérums des deux peptides.

### Purification de la protéine BARD1 clivée

Ces anticorps permettent aux auteurs de l'invention de purifier par chromatographie d'affinité la protéine tronquée soit à partir de corps apoptotiques de cellules tumorales soit à partir de la BARD1 entière produite et clivée *in vitro* comme décrit plus haut.

La purification est réalisée par des techniques standard de chromatographie d'affinité. Les fractions purifiées sont identifiées par western blot avec les anticorps produits.

### Exemple 4 :

### Vaccination des rats avec la protéine BARD1 tronquée

### Matériel et Méthodes

Deux groupes de rats BDIX ont reçu trois injections hebdomadaires en intraplantaire de 100 *µ*g de fragment F1 (acides aminés 460-611) de BARD1 ou d'une protéine contrôle (la fucosyl transférase) purifiée dans les mêmes conditions que le fragment F1. Les protéines étaient émulsifiées dans 100 µl d'adjuvant complet de Freund. Deux semaines après la demière immunisation, les cellules tumorales coliques de rat (PROb, 50 x 10³ /rat) sont injectées par voie sous-cutanée et le volume des tumeurs PROb est estimé.

### Résultat

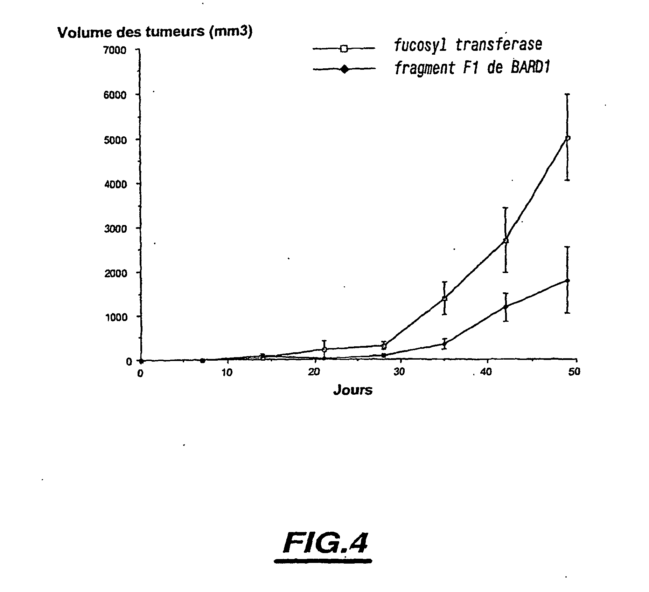

On observe, un ralentissement de la croissance tumorale démontrant ainsi l'effet protecteur d'une vaccination par un fragment de BARD1 issu de la forme 67 kD (Fig 4) (chaque point représente la moyenne des volumes des tumeurs mesurées sur 6 rats avec l'écart type).

### REFERENCES BIBLIOGRAPHIQUES

- Benoit et *al.,* PNAS USA, 79, 917-921 (1982).
- Boisteau et al, Apoptosis induced by sodium butyrate treatment increases immnogenicity of a rat colon tumor cell line. Apoptosis, *2:* 403-412, 1997.
- Caignard et al, Interaction between two cellular subpopulations of a rat colonic carcinoma when inoculated to syngeneic host. lnt.J.Cancer., *36*: 273-279, 1985.
- DeMartino, G. N., and Croall D.E. Purification and characterization of a protein inhibitor of calcium-dependent protease from rat liver. Arch. Biochem. Biophys., *232*: 7-13-720, 1984.
- Gautier et al, Production and characterization of a monoclonal antibody specific for apoptotic bodies derived from several tumor cell lines. J.Immunol.Meth., *228*: 49-58, 1999.
- Irminger-Finger et al, In vitro repression of Brca1-associated RING domain gene, Bard1, induces phenotypic changes in mammary epithelial cells. J.Cell Biol., *143:* 1329-1339, 1998.
- Jin et al, Cell cycle-dependent colocalization of BARD1 and BRCA1 proteins in discrete nuclear domains. Proc.Natl.Acad.Sci.USA., *94*: 12075-12080, 1997.
- Köhler et Milstein, *Nature,* 256, 495-497, (1975).- Laemmli et al, Cleavage of structural proteins during the assembly of the head of bacteriophage T4. Nature, *277*: 680-685, 1970.
- Laemmli, U.K. Cleavage of structural proteins during the assembly of the head of bacteriophage T4, Nature, *277*:680-685, 1970.
- Thai, et al, Mutation in the BRCA1-associated RING domain (BARD1) gene in primary breast ovarian and uterine cancers. Human Mol. Gen., *7*: 195-202, 1998.
- Wu et al, Identification of a RING protein that can interact in vivo with the BRCA1 gene product. Nature Gen., *14*:430-440, 1996.

## Revendications

1. Polypeptide isolé qui possède un poids moléculaire d'environ 67 kD, mesurable par électrophorèse dans des conditions dénaturantes (SDS-PAGE), et dont la séquence est constituée par 505 à 525 acides aminés à partir de l'extrémité C-terminale de BARD1 humaine.

2. Acide nucléique isolé constitué par une séquence nucléotidique codant pour le polypeptide de la revendication 1.

3. Vecteur de clonage et/ou d'expression comprenant la séquence d'acide nucléique de la revendication 2.

4. Cellule hôte isolée transfectée avec un vecteur selon la revendication 3.

5. Procédé de production d'un polypeptide selon la revendication 1, dans lequel un vecteur d'expression selon la revendication 3 est transféré dans une cellule hôte qui est mise en culture dans des conditions permettant l'expression de ladite protéine.

6. Composition pharmaceutique comprenant un polypeptide selon la revendication 1 en association avec un véhicule pharmaceutiquement acceptable.

7. Composition pharmaceutique comprenant un acide nucléique selon la revendication 2 en association avec un véhicule pharmaceutiquement acceptable.

8. Utilisation d'une composition pharmaceutique selon la revendication 6 ou 7 pour la fabrication d'un médicament destiné au traitement des tumeurs.

9. Méthode *in vitro* de suivi de l'efficacité d'un traitement anti-cancéreux chez un patient traité avec des drogues pro-apoptotiques, dans laquelle on détecte dans un échantillon biologique des anticorps dirigés contre le Polypeptide tel que défini dans la revendication 1 ou ledit polypeptide lui-même.

## Claims

1. Isolated polypeptide which has a molecular weight of approximately 67 kD, measurable by electrophoresis under denaturing conditions (SDS-PAGE), and the sequence of which is composed of from 505 to 525 amino acids from the C-terminal end of human BARD1.

2. Isolated nucleic acid composed of a nucleotide sequence coding for the polypeptide of claim 1.

3. Cloning and/or expression vector comprising the nucleic acid sequence of claim 2.

4. Isolated host cell transfected with a vector according to claim 3.

5. Process for the production of a polypeptide according to claim 1, in which an expression vector according to claim 3 is transferred into a host cell, which is cultured under conditions permitting the expression of said protein.

6. Pharmaceutical composition comprising a polypeptide according to claim 1 in association with a pharmaceutically acceptable carrier.

7. Pharmaceutical composition comprising a nucleic acid according to claim 2 in association with a pharmaceutically acceptable carrier.

8. Use of a pharmaceutical composition according to claim 6 or 7 in the manufacture of a medicament for treating tumours.

9. *In vitro* method for monitoring the effectiveness of an anti-cancer treatment in a patient treated with pro-apoptotic drugs, in which antibodies directed against the polypeptide as defined in claim 1, or said polypeptide itself, is/are detected in a biological sample.

## Patentansprüche

1. Isoliertes Polypeptid, das ein durch Elektrophorese unter denaturierenden Bedingungen (SDS-PAGE) meßbares Molekulargewicht von ungefähr 67 kD besitzt und dessen Sequenz aus 505 bis 525 Aminosäuren ausgehend vom C-terminalen Ende des humanen BARD1 besteht.

2. Isolierte Nukleinsäure, die aus einer für das Polypeptid des Anspruchs 1 kodierenden Nukteotidsequenz gebildet wird.

3. Klonierungs- und/oder Expressionsvektor umfassend die Nukleinsäuresequenz des Anspruchs 2.

4. Isolierte Wirtszelle, die mit einem Vektor gemäß dem Anspruch 3 transfziert ist.

5. Verfahren zur Herstellung eines Polypeptids gemäß Anspruch 1, bei dem ein Expressionsvektor gemäß Anspruch 3 in eine Wirtszelle überführt wird, die unter die Expression des Proteins erlaubenden Bedingungen kultiviert wird.

6. Pharmazeutische Zusammensetzung, die ein Polypeptid gemäß Anspruch 1 zusammen mit einem pharmazeutisch annehmbaren Träger umfaßt..

7. Pharmazeutische Zusammensetzung, die eine Nukleinsäure gemäß Anspruch 2 zusammen mit einem pharmazeutisch annehmbaren Träger umfaßt.

8. Verwendung einer pharmazeutischen Zusammensetzung gemäß Anspruch 6 oder 7 zur Herstellung eines zur Behandlung von Tumoren bestimmten Arzneimittels.

9. In-vitro-Verfahren zum Überwachen der Wirksamkeit einer Antikrebsbehandlung bei einem mit proapoptotischen Wirkstoffen behandelten Patienten, bei dem in einer biologischen Probe gegen das wie Anspruch 1 definierte Polypeptid gerichtete Antikörper oder das Polypeptid selbst nachgewiesen werden.


### LISTE DE SEQUENCES

<110> INSERM
<120> Protéine BARD1 tronquée et ses applications diagnostiques et thérapeutiques
<130> BFF 00/0493
<140>
   <141>
<160> 4
<170> PatentIn Ver. 2.1
<210> 1
   <211> 777
   <212> PRT
   <213> Homo sapiens
<400> 1
<210> 2
   <211> 22
   <212> ADN
   <213> Séquence artificielle
<220>
   <223> Description de la séquence artificielle:oligonucléotide (amorce PCR)
<400> 2
   caccaatgcc ttatgctgga gc 22
<210> 3
   <211> 22
   <212> ADN
   <213> Séquence artificielle
<220>
   <223> Description de la séquence artificielle:oligonucléotide (amorce PCR)
<400> 3
   gaagtagtga ctcctgagaa gg 22
<210> 4
   <211> 24
   <212> ADN
   <213> Séquence artificielle
<220>
   <223> Description de la séquence artificielle:oligonucléotide (amorce PCR)
<400> 4
   tcagctgtca agaggaagca actc 24